(19) **Europäisches Patentamt / European Patent Office / Office européen des brevets**

(11) **EP 4 428 808 A1**

(12) **EUROPEAN PATENT APPLICATION**

(43) Date of publication:
**11.09.2024 Bulletin 2024/37**

(21) Application number: **24162444.4**

(22) Date of filing: **08.03.2024**

(51) International Patent Classification (IPC):
**G06T 5/60** $^{(2024.01)}$

(52) Cooperative Patent Classification (CPC):
**G06T 5/60;** G06T 2207/10081; G06T 2207/10116;
G06T 2207/10132; G06T 2207/20081;
G06T 2207/20084

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **10.03.2023 US 202318181635**
**01.03.2024 JP 2024031514**

(71) Applicant: **Canon Medical Systems Corporation**
**Tochigi 324-0036 (JP)**

(72) Inventors:
• **HU, Yi**
**Otawara-shi, 324-0036 (JP)**
• **LI, Shijie**
**Otawara-shi, 324-0036 (JP)**
• **BAUMGART, John**
**Otawara-shi, 324-0036 (JP)**
• **MANAK, Joseph**
**Otawara-shi, 324-0036 (JP)**
• **SHIRAISHI, Kunio**
**Otawara-shi, 324-0036 (JP)**
• **YOSHIDA, Saki**
**Otawara-shi, 324-0036 (JP)**

(74) Representative: **Marks & Clerk LLP**
**15 Fetter Lane**
**London EC4A 1BW (GB)**

(54) **MEDICAL IMAGE PROCESSING METHOD, X-RAY DIAGNOSIS APPARATUS, AND GENERATION METHOD OF TRAINED MODEL**

(57) A medical image processing method according to an embodiment includes inputting first medical image data (402) to a trained model (404, 504, 504a, 804); and outputting, from the trained model, a medical image having an image quality higher than an image quality of the first medical image data, wherein the trained model (404, 504, 504a, 804) was trained using contrastive learning using second medical image data (508) as input data, third medical image data (304, 308, 510) and fourth medical image data (302, 306, 506) as label data, the third medical image data (304, 308, 510) being negative label data having worse image quality than the fourth medical image data (302, 306, 506), and the fourth medical image data (302, 306, 506) being positive label data having better image quality than the second medical image data (508).

FIG.7A

**Description**

BACKGROUND

The "background" description provided herein is for the purpose of generally presenting the context of the disclosure. Work of the presently named inventors, to the extent it is described in this background section, as well as aspects of the description which may not otherwise qualify as prior art at the time of filing, are neither expressly or impliedly admitted as prior art against the present invention.

Various medical images are known to be collected using X-rays and ultrasound. Since noise and blurriness may occur in medical images due to various factors, technologies to appropriately reduce them and improve image quality are desired.

Deep learning has been successfully applied to image quality improvement tasks. Despite the success of deep learning algorithms, they are difficult to control and tend to fall into unwanted solutions, and in particular, they have a hard time in learning good image quality and specifically excluding unwanted image features. Thus, there is a need for a controllable deep-learning algorithm to specifically exclude unwanted image features.

Summary of Invention

A medical image processing method provided according to an aspect of the present invention comprises: inputting first medical image data to a trained model; and outputting, from the trained model, a medical image having an image quality higher than an image quality of the first medical image data, wherein the trained model was trained using contrastive learning using second medical image data as input data, third medical image data and fourth medical image data as label data, the third medical image data being negative label data having worse image quality than the fourth medical image data, and the fourth medical image data being positive label data having better image quality than the second medical image data.

The third medical image data may be medical image data with blurriness.

The fourth medical image data may be medical image data with less noise and blurriness than the second medical image data.

The second medical image data may be image data with noise and blurriness, and the fourth medical image data may be image data with less noise and less blurriness than the second medical image data.

The fourth medical image data may be a high-dose image and the third medical image data is a low-dose image.

The second medical image data may be data output from the trained model that has received an input of input image data, and the third medical image data may be data based on the input image data.

The third medical image data may be generated by adding simulated blurriness to the fourth medical image data.

The third medical image data may be an image with less noise and more blurriness than the input image data.

The third medical image data may be generated by adding simulated blurriness and noise to the fourth medical image data.

The trained model may be trained based on a total loss obtained by adding a positive loss based on the second medical image data and the fourth medical image data to a contrastive loss based on a loss based on the second medical image data and the third medical image data and a loss based on the second medical image data and the fourth medical image data.

The contrastive learning may use a negative loss function term to learn from unwanted negative images used for the third medical image data.

The contrastive learning may simultaneously use a negative loss term and a positive loss term.

The contrastive learning may include encoding positive images, images predicted by the trained model, and the unwanted negative images so as to increase or decrease a weight for specific features.

The encoding may include passing the images through a projection layer.

The contrastive learning may include training a discriminator on an inverse of the contrastive loss using, as input to the discriminator, positive images, images predicted by the trained model, and the negative label data.

An X-ray diagnosis apparatus provided according to an aspect of the present invention comprises processing circuitry configured to: input first medical image data to a trained model; and output, from the trained model, a medical image having an image quality higher than an image quality of the first medical image data, wherein the trained model was trained using contrastive learning using second medical image data as an input, and third medical image data and fourth medical image data as label data, the third medical image data being negative label data having worse image quality than the fourth medical image data, and the fourth medical image data being positive label data having better image quality than the second medical image data.

The processing circuitry may further be configured to receive, as the first medical image data, X-ray fluoroscopy image data from a sequence of fluoroscopy images obtained by an image collector.

The processing circuitry may further be configured to: remove, from the trained neural network, weighted connections that are below a predetermined value; and reduce a precision of the weighted connections of the trained neural network.

The processing circuitry may include multiple processors and an image preprocessor; the image preprocessor may be configured to divide the first medical image data into a plurality of patches of image data; and the multiple processors may be configured to, based on the trained model, receive a subset of the plurality of patches of image data and generate respective restored patches of image data.

A method of generating a trained model provided according to an aspect of the present invention comprises: acquiring first training image data, second training image data, the second training image data being unwanted negative image data having worse image quality than third training image data, and the third training image data, the third training image data being wanted positive image data having better image quality than the first training image data; and training the neural network model using contrastive learning using the first training image data as input data and the second and third training image data as label data, wherein the contrastive learning includes a negative loss term for the neural network model to learn from the unwanted negative image data and a positive loss term for the neural network model to learn from the wanted positive image data.

The contrastive learning may simultaneously use the negative loss term in combination with the positive loss term.

The contrastive learning may include encoding positive images, images predicted by the trained model, and the unwanted negative image data so as to increase or decrease a weight for specific features.

The encoding may include passing the predicted images through a projection layer.

The contrastive learning may include training a discriminator on an inverse of the contrastive loss using, as input to the discriminator, positive image data, an image predicted by the trained model, and the unwanted negative image data.

BRIEF DESCRIPTION OF THE DRAWINGS

FIG. 1 is a block diagram illustrating the exemplary configuration of an X-ray diagnosis apparatus according to a first embodiment;
FIG. 2 is a schematic of an implementation of a computed tomography (CT) scanner according to the first embodiment;
FIG. 3A is a flow diagram for a data preparation pipeline, according to the first embodiment;
FIG. 3B is a flow diagram for a data preparation pipeline according to the first embodiment;
FIG. 4A is a flow diagram of applying a neural network to an X-ray image, according to the first embodiment;
FIG. 4B is a flow diagram of applying the neural network to an X-ray image, according to the first embodiment;
FIG. 4C is a flow diagram of applying the neural network to an X-ray image, according to the first embodiment;
FIG. 5 is a flow diagram for a method of training a neural network by a contrastive loss that includes a positive loss and a negative loss, according to the first embodiment;
FIG. 6 is a flow diagram for a method of training a neural network by a contrastive loss that includes a fixed pretrained encoder, according to the first embodiment;
FIG. 7A is a flow diagram showing an application example of contrastive learning including encoding according to the first embodiment;
FIG. 7B is a diagram illustrating a configuration example of the neural network according to the first embodiment;
FIG. 7C shows image quality obtained from various types of image processing according to the first embodiment;
FIG. 8 is a flow diagram for deep learning-based image restoration, according to the first embodiment;
FIG. 9 is a flow diagram for deep learning-based image restoration that includes pruning and precision reduction for real-time inferencing, according to the first embodiment;
FIG. 10 is a flow diagram for deep learning-based image restoration that is implemented on multiple processors for real-time inferencing, according to the first embodiment;
FIG . 11 is a flow diagram for deep learning-based image restoration that is implemented on multiple processors for real-time inferencing, according to the first embodiment; and
FIG. 12 is a block diagram illustrating an example computer system for implementing the machine learning training and inference methods according to the first embodiment.

DETAILED DESCRIPTION

The following describes embodiments of a medical image processing method, an X-ray diagnosis apparatus, and a generation method of a trained model with reference to the accompanying drawings.

In the drawings, like reference numerals designate identical or corresponding parts throughout the several views. Further, as used herein, the words "a," "an" and the like generally carry a meaning of "one or more," unless stated otherwise.

A first embodiment describes a case in which X-ray images collected using X-rays are subjected to processing.

X-ray imaging techniques utilize the transimission process of X-ray through an object, where X-ray photons that are not absorbed by the object reach a receptor form a shadow of the object. The resulting static image is acquired on a receptor, which is typically a FPD (Flat Panel Detector). The X-ray images acquired can be used in two ways. (1) The raw 2D projections can be used in diagnostics (e.g. radiographs, mammography etc.) or surgical guidance (e.g. fluoroscopy, digital angiography etc.) directly. (2) The raw 2D projections taken at different angles can be used to reconstruction a 3D volume of the objects (e.g. computed tomography, cone-beam computed tomography, tomosynthesis etc.).

The image quality of X-ray images is limited by the relatively smaller number of photons reaching the receptor during the relatively short exposure time. The resolution of X-ray images is limited by the blurriness from scintillator, focal spot and geometry, subsequently, the image quality of fluoroscopy sequences suffers from noise and blurriness problems.

For example, the image quality of 2D X-ray images (e.g., fluoroscopy, X-ray images, cone-beam CT/CT projection etc.) and reconstructed 3D images (e.g., cone-beam CT or CT volumes, etc.) suffers from noise and blurriness problems. Projection according to the present disclosure relates to a scan that produces a 2D image. Reconstruction according to the present disclosure relates to creation of a 3D image from several scans from different angles.

As an example of noise and blurriness, devices used in interventional surgeries (e.g., stents, guidewires, etc.) can be made visible using X-ray fluoroscopic images. However, image quality of fluoroscopic sequences suffers from noise and blurriness. Deep learning networks have improved in their capability of image classification, as well as have been shown to be effective in reducing noise and blurriness, but still have a tendency to fall into unwanted solutions during training. For example, an image that has poor image quality can be mistakenly considered as being of good quality during training. In particular, it is difficult to control a deep learning algorithm to improve image quality. The disclosure relates to controllable deep learning to specifically exclude unwanted image appearance. In one embodiment, the deep learning excludes unwanted image appearance by using contrastive learning. The disclosure also provides a data preparation pipeline to collect or simulate unwanted images.

Radiation according to the present disclosure can include not only $\alpha$-rays, $\beta$-rays, and $\gamma$-rays that are beams generated by particles (including photons) emitted by radioactive decay, but also beams having equal or more energy, for example, X-rays, particle rays, and cosmic rays.

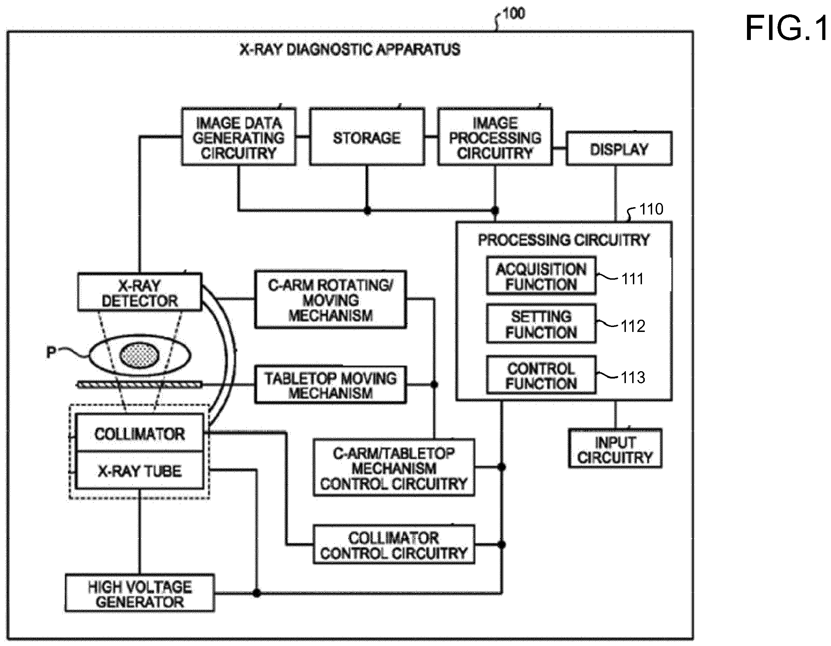

In one embodiment, an X-ray diagnostic apparatus can be an X-ray diagnostic apparatus with a C-arm. FIG. 1 is a block diagram illustrating an exemplary configuration of the X-ray diagnosis apparatus 100.

For example, the X-ray diagnosis apparatus 100 includes imaging mechanisms such as an X-ray detector, an X-ray tube, a collimator, a high-voltage generator, a C-arm rotation/moving mechanism, a table top moving mechanism, a C-arm/table top mechanism control circuit, and a collimator control circuit. The X-ray diagnosis apparatus 100 operates these imaging mechanisms under the control of processing circuitry 110 to perform imaging of an object P. That is, the X-ray diagnosis apparatus 100 emits X-rays from the X-ray tube to the object P and collects detection signals by detecting X-rays transmitted through the object P with the X-ray detector.

The X-ray diagnosis apparatus 100 also includes an image data generation circuit and an image processing circuit. The image data generation circuit generates an X-ray image based on the detection signals output from the X-ray detector and stores them in a storage. The image processing circuit also performs certain processing on the generated X-ray image. A display also shows the X-ray image after image processing by the image processing circuit and a graphical user interface (GUI) for receiving various instructions and settings from a user via an input circuit.

The processing circuitry 110 controls the operation of the entire X-ray diagnosis apparatus 100 by executing an acquisition function 111, a setting function 112, and a control function 113.

The acquisition function 111 acquires a trained model and stores it in the storage. For example, the acquisition function 111 acquires a trained model that is functionalized to output a medical image with high image quality by performing neural network training.

For example, the acquisition function 111 acquires, as training data, second medical image data, third medical image data, which is unwanted negative example image data with lower image quality than that of the second medical image data, and fourth medical image data, which is wanted positive example image data with higher image quality than that of the second medical image data. For example, the acquisition function 111 acquires, as training data, X-ray images collected by the X-ray diagnosis apparatus 100 or from other apparatuses via a network (not shown) and stores them in a storage circuit 111. The acquisition function 111 also trains a neural network model using contrastive learning, which uses the second medical image data as input data and the third medical image data and the fourth medical image data as label data. Here, the contrastive learning includes a negative loss term for the neural network model to learn from the unwanted negative example image data and a positive loss term for the neural network model to learn from the wanted positive example image data. That is, the acquisition function 111 can use contrastive learning to perform generation processing of a trained model (learning phase). Details of the learning phase are described later. Alternatively, the acquisition function 111 may acquire a trained model from another apparatus via a network (not shown).

The setting function 112 sets the conditions for imaging X-ray images and image processing, and the like. For example, the setting function 112 sets various conditions according to operations received from the user via the input circuit. Setting of conditions may be done partially or fully automatically based on information such as a site of imaging and a case.

The control function 113 performs imaging on the object P by controlling the operation of the imaging mechanisms. For example, the C-arm/table top mechanism control circuit drives the C-arm and a table (bed) on which the object P is placed under the control of the control function 113 to control an imaging position and an imaging angle. The collimator control circuit drives aperture blades included in the collimator under the control of the control function 113 to narrow an X-ray irradiation range. The high voltage generator supplies high voltage to generate X-rays to the X-ray tube under the control of the control function 113. With this operation, X-rays are emitted to the object P, the X-rays transmitted through the object P are detected by the X-ray detector, and detection signals can be collected.

The control function 113 also inputs first medical image data to the trained model and executes a process to output a medical image with higher image quality than that of the first medical image data from the trained model. That is, the control function 113 can perform image processing (inference phase) using the trained model described later. Details of the inference phase are described later.

The X-ray diagnosis apparatus 100 shown in FIG. 1 is merely an example, and various variations are possible. For example, the X-ray diagnosis apparatus 100 is not limited to a C-arm type X-ray diagnosis apparatus in which an X-ray tube and an X-ray detector are held by a C-arm. Alternatively, the X-ray images according to the present embodiment may be collected by an X-ray computed tomography (CT) apparatus. An X-ray CT apparatus is also described as a CT scanner.

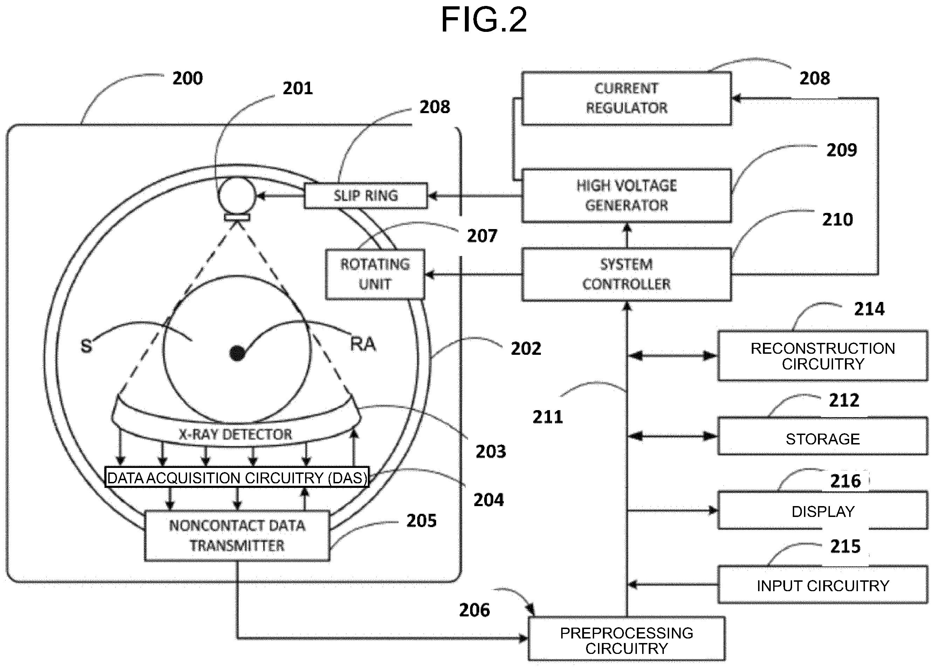

FIG. 2 is a schematic of an implementation of a computed tomography (CT) scanner. As shown in FIG. 2, a radiography gantry 200 is illustrated from a side view and further includes an X-ray tube 201, an annular frame 202, and a multi-row or two-dimensional-array-type X-ray detector 203. The X-ray tube 201 and X-ray detector 203 are diametrically mounted across an object OBJ on the annular frame 202, which is rotatably supported around a rotation axis RA. A rotating unit 207 rotates the annular frame 202. For example, the rotating unit 207 can rotate the annular frame 202 at a high speed, such as 0.4 sec/rotation. While the annular frame 202 is rotating, the object OBJ is being moved along the axis RA into or out of the illustrated page.

The embodiment of an X-ray computed tomography (CT) apparatus according to the present inventions will be described below with reference to the views of the accompanying drawing. Note that X-ray CT apparatuses include various types of apparatuses, e.g., a rotate/rotate-type apparatus in which an X-ray tube and X-ray detector rotate together around an object to be examined, and a stationary/rotate-type apparatus in which many detection elements are arrayed in the form of a ring or plane, and only an X-ray tube rotates around an object to be examined. The present embodiment can be applied to either type. In this case, the rotate/rotate type, which is currently the mainstream, will be exemplified.

The multi-slice X-ray CT apparatus further includes a high voltage generator 209 that generates a tube voltage applied to the X-ray tube 201 through a slip ring 208 so that the X-ray tube 201 generates X-rays. The X-rays are emitted towards the object OBJ, whose cross-sectional area is represented by a circle. For example, the X-ray tube 201 having an average X-ray energy during a first scan that is less than an average X-ray energy during a second scan. Thus, two or more scans can be obtained corresponding to different X-ray energies. The X-ray detector 203 is located at an opposite side from the X-ray tube 201 across the object OBJ for detecting the emitted X-rays that have transmitted through the object OBJ. The X-ray detector 203 further includes individual detector elements or units.

The CT apparatus further includes other devices for processing the detected signals from X-ray detector 203. A data acquisition circuitry or a Data Acquisition System (DAS) 204 converts a signal output from the X-ray detector 203 for each channel into a voltage signal, amplifies the signal, and further converts the signal into a digital signal. The X-ray detector 203 and the DAS 204 are configured to handle a predetermined total number of projections per rotation (TPPR).

The above-described data is sent to a preprocessing circuitry 206, which is housed in a console outside the radiography gantry 200 through a non-contact data transmitter 205. The preprocessing circuitry 206 performs certain corrections, such as sensitivity correction on the raw data. A storage 212 stores the resultant data, which is also called projection data at a stage immediately before reconstruction processing. The storage 212 is connected to a system controller 210 through a data/control bus 211, together with reconstruction circuitry 214, input circuitry 215, and display 216. The system controller 210 controls a current regulator 213 that limits the current to a level sufficient for driving the CT system.

The detectors are rotated and/or fixed with respect to the patient among various generations of the CT scanner systems. In one implementation, the above-described CT system can be an example of a combined third-generation geometry and fourth-generation geometry system. In the third-generation system, the X-ray tube 201 and the X-ray detector 203 are diametrically mounted on the annular frame 202 and are rotated around the object OBJ as the annular frame 202 is rotated about the rotation axis RA. In the fourth-generation geometry system, the detectors are fixedly placed around the patient and an X-ray tube rotates around the patient. In an alternative embodiment, the radiography gantry 200 has multiple detectors arranged on the annular frame 202, which is supported by a C-arm and a stand.

The storage 212 can store the measurement value representative of the irradiance of the X-rays at the X-ray detector 203. The storage 212 also stores a computer program for the circuits included in the X-ray CT apparatus shown in FIG. 2 to implement the functions of the circuits.

The reconstruction circuitry 214 can perform reconstruction processing based on the projection data, image processing on the reconstructed image, and generation processing of an image for display. The reconstruction circuitry 214 can also perform the generation processing of a trained model (learning phase) similarly to the acquisition function 111. The reconstruction circuitry 214 can also perform image processing (inference phase) using the trained model similarly to the control function 113.

The pre-reconstruction processing of the projection data performed by the preprocessing circuitry 206 can include correcting for detector calibrations, detector nonlinearities, and polar effects, for example.

The reconstruction circuitry 214 can include a CPU (processing circuitry) that can be implemented as discrete logic gates, as an Application Specific Integrated Circuit (ASIC), a Field Programmable Gate Array (FPGA) or other Complex Programmable Logic Device (CPLD). An FPGA or CPLD implementation may be coded in VHDL, Verilog, or any other hardware description language and the code may be stored in an electronic memory directly within the FPGA or CPLD, or as a separate electronic memory. Further, the storage 212 can be non-volatile, such as ROM, EPROM, EEPROM or FLASH memory. The storage 212 can also be volatile, such as static or dynamic RAM, and a processor, such as a microcontroller or microprocessor, can be provided to manage the electronic memory as well as the interaction between the FPGA or CPLD and the memory.

Alternatively, the CPU in the reconstruction circuitry 214 can execute a computer program including a set of computer-readable instructions that perform the functions described herein, the program being stored in any of the above-described non-transitory electronic memories and/or a hard disk drive, CD, DVD, FLASH drive or any other known storage media. Further, the computer-readable instructions may be provided as a utility application, background daemon, or component of an operating system, or combination thereof, executing in conjunction with a processor, such as a Xenon processor from Intel of America or an Opteron processor from AMD of America and an operating system, such as Microsoft VISTA, UNIX, Solaris, LINUX, Apple, MAC-OS and other operating systems known to those skilled in the art. Further, CPU can be implemented as multiple processors cooperatively working in parallel to perform the instructions.

In one implementation, the reconstructed images can be displayed on a display 216. The display 216 can be an LCD display, CRT display, plasma display, OLED, LED or any other display known in the art.

The storage 212 can be a hard disk drive, CD-ROM drive, DVD drive, FLASH drive, RAM, ROM or any other electronic storage known in the art.

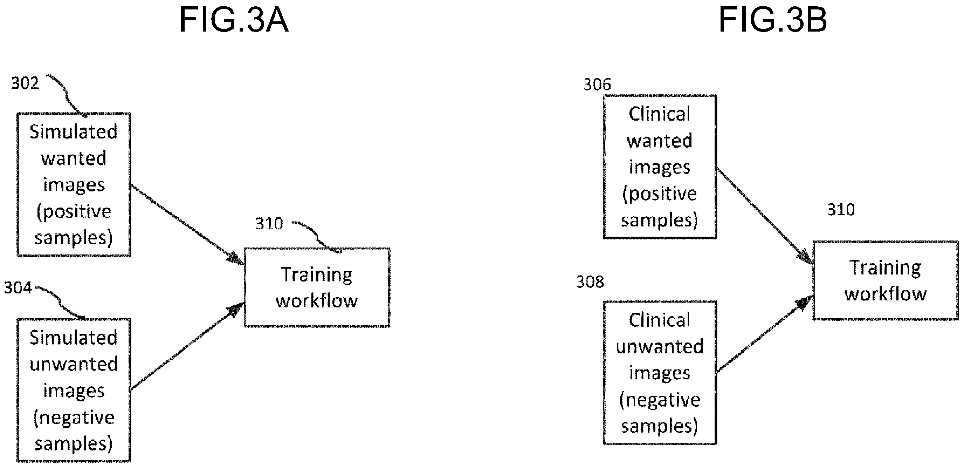

FIGs. 3A and 3B are flow diagrams for a data preparation pipeline, in accordance with an exemplary aspect of the present disclosure. Medical images are often blurry and noisy making them difficult to interpret. For example, fluoroscopic images tend to show blurry edges and overly unclear textures, due in part to noise. Conventional positive-loss-based deep-learning image restoration algorithms tend to over-smooth images, due in part to accepting unwanted images as being positive. In other words, a trained model generated by the conventional method tends to have residual blurriness, even though noise can be removed. In contrast, medical images, such as fluoroscopic images, of high image quality are those that have clear edges and accurate texture.

In a data preparation stage, unwanted images (negative samples) are prepared as well as positive images (positive samples). In development of disclosed embodiments, it has been determined that increasing the number of, and explicit rejection of, unwanted images results in medical images that are more accurately smoothed and have clearer edges. Unwanted images 308 can be selected from actual clinical images, but unwanted images 304 can also be obtained through simulation.

For purposes of this disclosure, unwanted images (negative samples) are images that have general blurriness in at least one of edges and texture, and/or are images with at least one artifact. Unwanted images 304 can be obtained through simulation by simulating a blurred image from a good quality image, adding arbitrary artifacts to the good quality image, as well as adding noise to reduce image quality. Blurriness is simulated by adjusting texture and softening edges. Artifacts can be extracted from clinical images and incorporated into simulated images. Artifacts can also be generated by the simulation based on other known artifacts or may be manually produced and incorporated into the simulation.

For purposes of this disclosure, a wanted image (positive sample) is one that is substantially free of artifacts and blurriness such that edges and texture are visually clear and accurate. Wanted images 302 can also be obtained through simulation, as well as selected from actual clinical images 306. One approach to obtaining wanted images is to start with a good quality image and simulate movement of the good quality image. Another approach to obtaining wanted images is to generate different views of a good quality image. In this disclosure, image quality can be used to distinguish wanted images from unwanted images. Positive samples are wanted images having better image quality than unwanted images (negative samples) in terms of quantitative measurements (e.g., less noise, less blurriness, higher resolution, artifact-free, etc.). For example, in an embodiment, an image with lower image quality will have at least one of: increased noise, blurriness or artifacts, when compared to an image with higher image quality.

However, other criteria can be used to determine wanted and unwanted images used for training.

A deep learning network 310 is trained using combinations of simulated and clinical images, simulated images only, or clinical images only, depending on the availability of positive and unwanted images. In one embodiment, the deep learning network 310 is trained with at least one unwanted image. It is preferred that each unwanted (negative) image in a training set have at least one corresponding positive image.

The simulated wanted image (positive sample) shown in FIG. 3A and the wanted clinical image (positive sample) shown in FIG. 3B are examples of the fourth medical image data. The fourth medical image data is medical image data with higher image quality than that of the second medical image data described later, and is used as positive label data in contrastive learning. For example, the fourth medical image data is medical image data with less noise and blurriness than those of the second medical image data. As described above, the fourth medical image data may be generated by simulation or may be a clinical image. In the embodiment, clinical images mean images collected by a modality apparatus such as the X-ray diagnosis apparatus 100 in FIG. 1 or the X-ray CT apparatus in FIG. 2. The imaging subject in a clinical image is not limited to an object, but can also be a phantom for imaging.

The simulated unwanted image (negative sample) shown in FIG. 3A and the unwanted clinical image (negative sample) shown in FIG. 3B are examples of the third medical image data. The third medical image data is medical image data with lower image quality than that of the fourth medical image data and is used as negative label data in contrastive learning. For example, the third medical image data is medical image data with blurriness. For example, the third medical image data has a higher degree of blurriness than the fourth medical image data. As described above, the third medical image data may be generated by simulation or may be a clinical image.

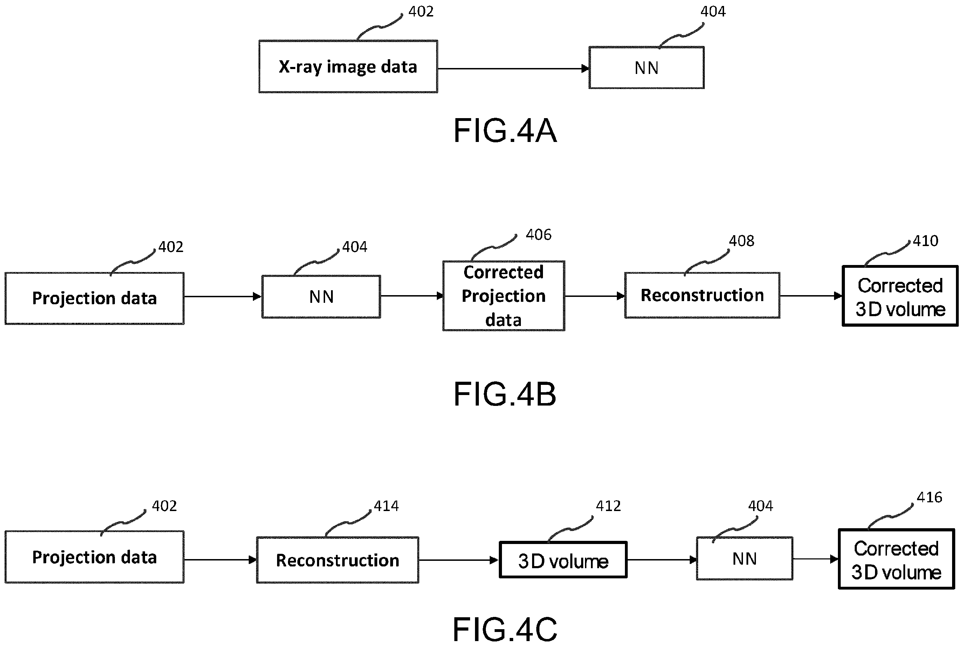

FIGs. 4A, 4B, and 4C are flow diagrams of applying neural networks to 2D medical images and 3D medical images, in accordance with an exemplary aspect of the disclosure. In the embodiment, various medical images collected using X-rays are collectively referred to simply as X-ray images. For example, examples of X-ray images include two-dimensional X-ray images collected by X-ray diagnosis apparatuses, three-dimensional X-ray images reconstructed from two-dimensional X-ray images, projection data collected by X-ray CT apparatuses, and CT images reconstructed from projection data.

In FIG. 4A, in the case of 2D medical images, the neural network 404 is applied directly to X-ray image data 402, which is projection data. For purposes of this disclosure, X-ray images are reconstructed from a number of projections that are acquired as an X-ray tube rotates through 360° around the object (patient). The neural network 404 is an example of a trained model and is trained using contrastive learning. In FIG. 4A, the X-ray image data 402 is an example of the first medical image data, and the neural network 404 outputs medical images with higher image quality than that of the X-ray image data 402.

In FIG. 4B, for 3D medical images, the neural network 404 is applied to the projection data 402 to obtain corrected projection data 406, before reconstruction 408, to obtain a corrected 3D volume 410. In FIG. 4B, the projection data 402 is an example of the first medical image data, and the corrected projection data 406 is an example of a medical image with higher image quality than that of the first medical image data.

In FIG. 4C, in another embodiment for 3D medical images, the neural network 404 is applied to the reconstruction 3D volume 412 after reconstruction 414, to obtain corrected 3D volume 416. For purposes of this disclosure, cone-beam computed tomography (CBCT) is a radiographic imaging method for obtaining three-dimensional (3D) imaging of hard tissue structures. In FIG. 4C, the three-dimensional volume 412 is an example of the first medical image data, and the corrected three-dimensional volume 416 is an example of a medical image with higher image quality than that of the first medical image data. The neural network 404 may be applied to both the projection data and the reconstructed three-dimensional volume, not one of them.

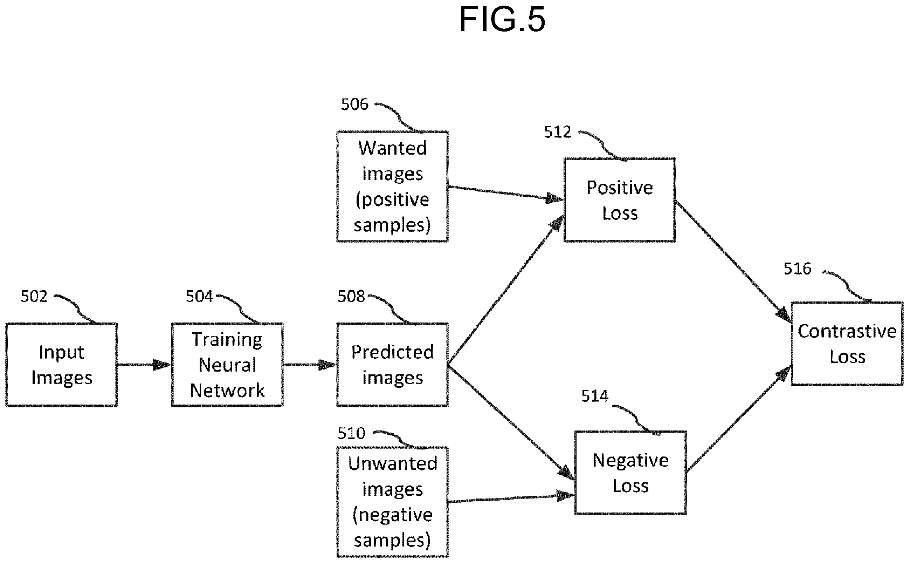

FIG. 5 is a flow diagram for a method of training a neural network by contrastive loss that includes a positive loss and a negative loss, in accordance with an exemplary aspect of the present disclosure. All images of a training set, including wanted images (positive samples) and unwanted images (negative samples) with poor image quality, are input for training a neural network 504. Images can be input for training one image at a time, or as a batch (or mini-batch) of training data. The neural network 504 is an example of a neural network model. By training the neural network 504, a trained model can be generated.

The neural network 504 can be any neural network configured for image restoration, where the input is an image and the output is an image having been generated by the neural network as a predicted image 508 with improved image quality. A set of wanted images (positive samples) 506 and a set of unwanted images (negative samples) 510 are used in the calculation of a contrastive loss 516. The contrastive loss is fed back to update the neural network 504 during training. The neural network 504 is trained by contrastive learning.

For example, in FIG. 5, an input image 502 is input to the neural network 504 and the predicted image 508 is output. A positive loss 512 is also acquired based on the wanted image (positive sample) 506 and the predicted image 508. A negative loss 514 is also acquired based on the unwanted image (negative sample) 510 and the predicted image 508. The contrastive loss 516 is acquired based on the positive loss 512 and the negative loss 514, and the neural network 504 is updated based on the contrastive loss 516. That is, the neural network 504 shown in FIG. 5 is trained by contrastive learning.

For example, the contrastive loss term includes a negative loss term based on the predicted output 508 of the neural network 504 and unwanted images 510, as well as a positive loss term based on a difference between the predicted output 508 and wanted images 506. That is, the contrastive learning simultaneously uses the negative loss term and the positive loss term. For example, the contrastive learning uses the negative loss function term to learn from unwanted negative images used for the third medical image data.

The contrastive loss term can also include a term that is the difference between the input image 502 and the predicted image 508. For example, in FIG. 5, the input image 502 may be used as the unwanted image (negative sample) 510 and the negative loss 514 may be acquired based on the input image 502 and the predicted image 508.

The predicted image 508 shown in FIG. 5 is an example of the second medical image data. The unwanted image (negative sample) 510 is an example of the third medical image data. The wanted image (positive sample) 506 is an example of the fourth medical image data. As described above, the neural network 504 is trained using contrastive learning, which uses the second medical image data as input data and the third medical image data and the fourth medical image data as label data.

The second medical image data is also described as first training image data. The third medical image data is also described as second training image data. The fourth medical image data is also described as third training image data.

As described above, the third medical image data and the fourth medical image data may be generated by simulation or may be clinical images. For example, the wanted image (positive sample) 506 shown in FIG. 5 is a high-dose image collected under high-dose X-ray conditions, and the unwanted image (negative sample) 510 may be a low-dose image collected under low-dose X-ray conditions. To give an example, a fluoroscopic image collected under low-dose X-ray conditions can be used as the unwanted image (negative sample) 510, and a DA image (digital angiographic image) collected under high-dose X-ray conditions compared to a fluoroscopic image can be used as the wanted image (positive sample) 506.

As described above, the third medical image data is negative label data with lower image quality than that of the fourth medical image data. For example, the third medical image data is generated to contain at least more blurriness than that of the fourth medical image data. For example, the unwanted image (negative sample) 510 shown in FIG. 5 is generated by adding simulated blurriness to the wanted image (positive sample) 506. In addition, for example, the unwanted image (negative sample) 510 is generated by adding simulated blurriness and noise to the wanted image (positive sample) 506.

In addition, at a stage when the neural network 504 has not been sufficiently trained, the predicted image 508, which is the output from the neural network 504, will contain noise and blurriness. There is less noise and blurriness in the wanted image (positive sample) 506 when compared to the predicted image 508. That is, the second medical image data may be image data with noise and blurriness, and the fourth medical image data is image data with less noise and less blurriness than the second medical image data.

The input image 502 shown in FIG. 5 is an example of input image data. As with the case of the third medical image data and the fourth medical image data, input image data may be generated by simulation or may be clinical images. For example, the wanted image (positive sample) 506 shown in FIG. 5 is a high-dose image, while the unwanted image (negative sample) 510 and the input image 502 may be low-dose images.

The unwanted image (negative sample) 510 may be data based on the input image 502. For example, the unwanted image (negative sample) 510 may be the input image 502 itself or an image obtained by improving or reducing the image quality of the input image 502. That is, in FIG. 5, the predicted image 508 and the unwanted image (negative sample) 510 may be acquired based on the input image 502. In other words, the second medical image data may be data output from the neural network 504 that has received an input of input image data, and the third medical image data may be data based on the input image data.

A difference function d( ) can be a Mean Absolute Error (MAE) or a Mean Squared Error (MSE), which is also referred to as Root Mean-Squared Error. These errors represent the differences between the predicted values (values predicted by the neural network) and the actual values. In one embodiment, the contrastive loss is determined by the following equation (1) .

$$Contrastive\ Loss = \frac{d(Y,\ P)}{d(Y,\ N)} + \frac{d(Y,\ P)}{d(Y,\ X)} \qquad \cdots (1)$$

In equation (1), d( ) is MAE or MSE, Y is the output of the neural network, P refers to the positive samples, N refers to the negative samples, and X is the input image.

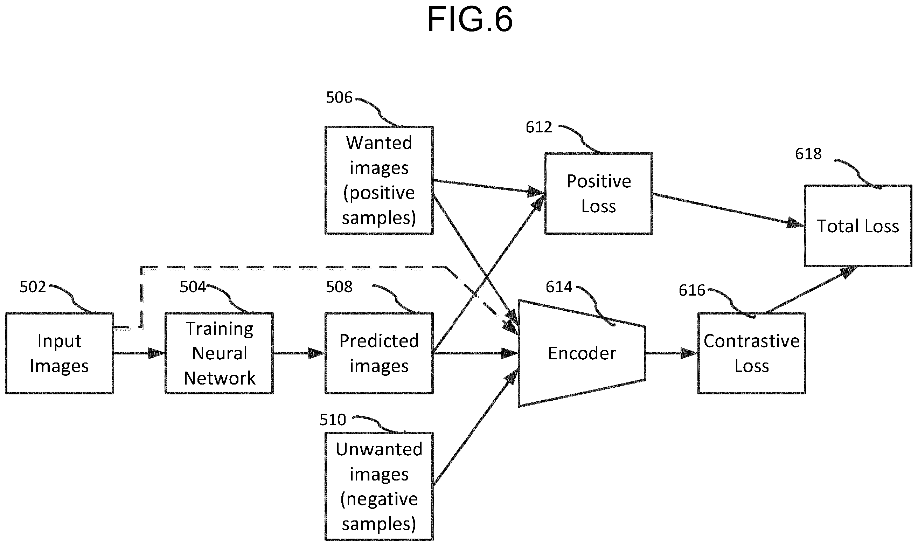

FIG. 6 is a flow diagram for a method of training a neural network by a contrastive loss method that includes a fixed pretrained encoder, in accordance with an exemplary aspect of the present disclosure. Using the encoder can highlight differences between images and streamline the acquisition of contrastive losses. For example, the encoder may apply image processing to highlight features of each input image or align standards and statistics across images to clarify differences between images and facilitate computation of contrastive losses.

The encoder may be a fully connected network with a number of layers L. In FIG. 6, the total loss function 618 includes a fixed pretrained encoder 614 to encode a wanted image 506, a predicted image 508, and an unwanted image 510 into respective encoded images. A positive loss 612 is added to a contrastive loss 616 to generate the total loss 618. The contrastive loss can be computed, for example, by the following equation (2).

$$Contrastive\ Loss = \sum_{l=1}^{L} w_l \frac{d(E(Y)^l,\ E(P)^l)}{d(E(Y)^l,\ E(N)^l)} \qquad \cdots (2)$$

In equation (2), E is a fixed pretrained encoder, d( ) is MAE or MSE, Y is the output of the neural network, P refers to the positive samples, N refers to the negative samples, X is the input image, L refers to the number of intermediate layers in the encoder, and wl is a weight for each intermediate layer.

In one embodiment, specific features in one or more unwanted images can be emphasized using a mask.

In one embodiment, the encoder 614 is configured with an additional projection layer and a normalization layer. The projection layer can receive inputs from a wanted image 506, a predicted image 508, and an unwanted image 510, which are mapped to a vector space of a reduced dimension. That is, encoding according to the embodiment includes passing the images through a projection layer. A projection layer is typically a small neural network, e.g., an MLP (Multi-Layer Perceptron) with one hidden layer, that is used to map the representations from the base encoder to a reduced dimensional latent space.

The normalization layer normalizes the input across the features. Normalization is used for training the neural network so that the different features are on a similar scale.

In one embodiment, the difference is determined as an exponentiation of a first term "a" multiplied by a second term "b", divided by a constant "tau".

The contrastive loss 616 is determined using results from the encoder 614. The positive loss 612 is determined using a difference between the predicted image 508 and the wanted image 506. In another embodiment, the contrastive loss can be computed by the following equation (3) and equation (4) .

$$Contrastive\ Loss = \sum_{l=1}^{L} w_l \frac{d(E(Y)^l,\ E(P)^l)}{d(E(Y)^l,\ E(N)^l) + d(E(Y)^l,\ E(P)^l)} \qquad \cdots (3)$$

$$d(a,\ b) := exp(a \cdot b/\tau) \qquad \cdots (4)$$

In equations (3) and (4), E is a fixed pretrained encoder with an additional projection layer and normalization layer, d(a, b) is a dot product similarity function where $\tau$ is a constant, referred to as a temperature scalar, Y is the output of the neural network, P refers to the positive samples, N refers to the negative samples, X is the input image, L refers to the number of intermediate layers in the encoder, and wl is a weight for each intermediate layer. In one embodiment, a contrastive loss method includes a fixed pretrained encoder and a logarithmic function. In the one embodiment, the encoder 614 of the total loss 618 applies a weighted logarithm to each intermediate layer of an encoder 614 of L layers. The logarithmic function helps to keep the weighted values low. The contrastive loss 616 is determined using results from the encoder 614. The positive loss 612 is determined using a difference between the predicted image 508 and the wanted image 506. In one embodiment, the contrastive loss is computed by the following equation (5).

$$Contrastive\ Loss = -\sum_{l=1}^{L} w_l log\left(\frac{d(E(Y)^l,\ E(P)^l)}{d(E(Y)^l,\ E(N)^l)} + \frac{d(E(Y)^l,\ E(P)^l)}{d(E(Y)^l,\ E(X)^l)}\right) \quad \cdots (5)$$

In equation (5), E is a fixed pretrained encoder, d( ) is MAE or MSE, Y is the output of the neural network, P refers to the positive samples, N refers to the negative samples, X is the input image, L refers to the number of intermediate layers in the encoder, and wl is a weight for each intermediate layer.

In one embodiment, a contrastive loss method includes a fixed pretrained encoder with an additional projection and normalization and a logarithmic function. The total loss 618 includes an encoder 614. The encoder 614 is configured with an additional projection layer and a normalization layer. The total loss 618 includes a difference that is determined based on an exponential of a term "a" multiplied by a term "b", over a constant "tau." The contrastive loss 616 is determined using results from the encoder 614. The positive loss 612 is determined using a difference between the predicted image 508 and the wanted image 506. In one embodiment, the contrastive loss is computed by the following equation (6) and equation (7) .

*Contrastive Loss*

$$= -\sum_{l=1}^{L} w_l log \frac{d(E(Y)^l,\ E(P)^l)}{d(E(Y)^l,\ E(N)^l) + d(E(Y)^l,\ E(P)^l) + d(E(Y)^l,\ E(X)^l)} \quad \cdots (6)$$

$$d(\boldsymbol{a},\ \boldsymbol{b}) := exp(\boldsymbol{a} \cdot \boldsymbol{b}/\tau) \quad \cdots (7)$$

In equations (6) and (7), E is a fixed pretrained encoder with an additional projection layer and normalization layer, d(a, b) is a dot product similarity function where $\tau$ is a constant, referred to as a temperature scalar, Y is the output of the neural network, P refers to the positive samples, N refers to the negative samples, X is the input image, L refers to the number of intermediate layers in the encoder, and wl is a weight for each intermediate layer.

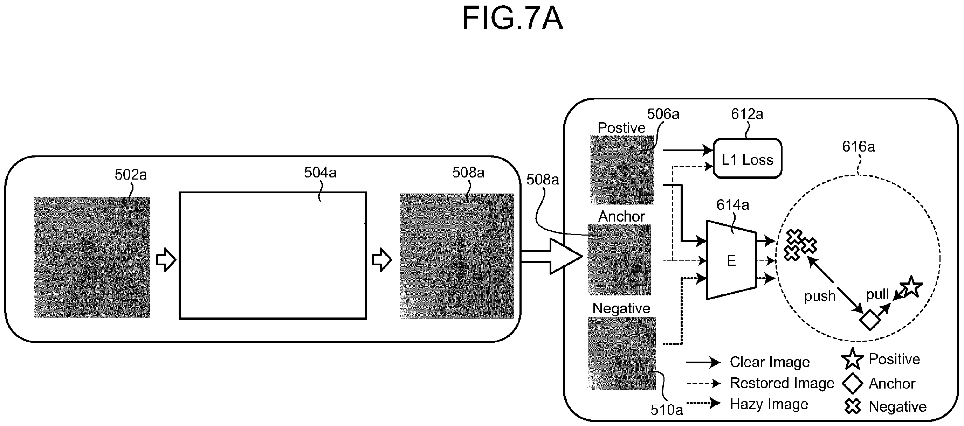

Examples of contrastive learning including encoding have been described above with reference to FIG. 6. Next, more specific application examples of contrastive learning including encoding will be described with reference to FIGs. 7A to 7C.

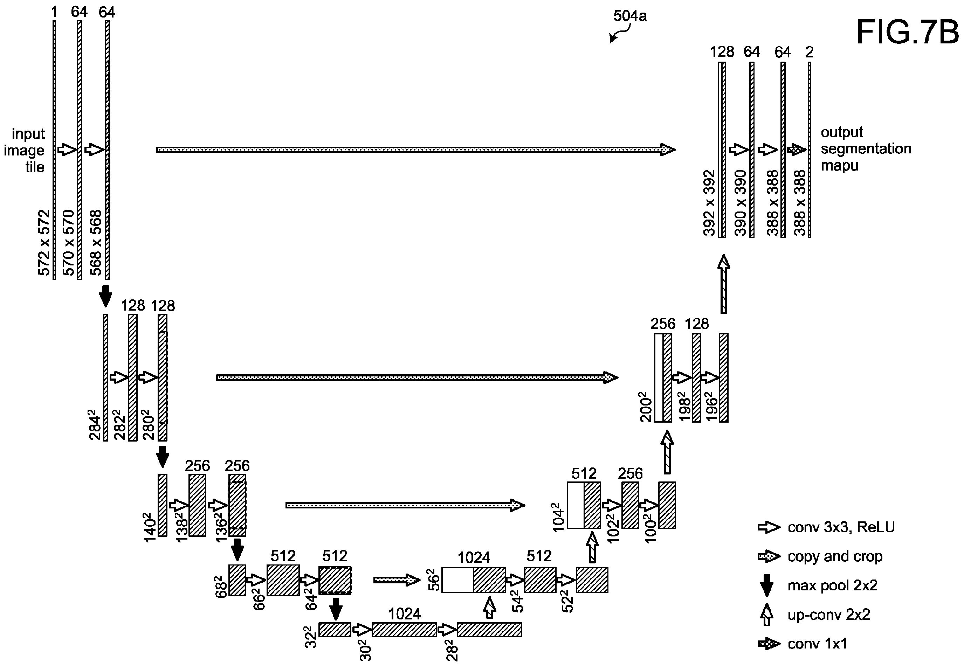

In FIG. 7A, an X-ray image 502a is an example of the input image 502 shown in FIG. 6. A U-net 504a is an example of the neural network 504 shown in FIG. 6. The U-net 504a is a type of convolutional neural network that uses semantic segmentation technology. The series of convolution, feature extraction, and other processes performed in the U-net 504a can be illustrated as a "U"-shaped structure, as shown in FIG. 7B. The structure of the U-net 504a shown in FIG. 7B and the numbers in the drawing are only examples and do not limit the embodiment.

In FIG. 7A, an X-ray image 508a is data output from the U-net 504a that has received the input of the X-ray image 502a and is also an example of the predicted image 508 shown in FIG. 6. The X-ray image 508a is used as "Anchor" data in training the U-net 504a using contrastive learning. That is, the X-ray image 508a is input as input data (Restored Image) to be restored in the computation of L1Loss 612a and encoding processing by an encoder 614a.

FIG. 7A is a diagram showing a series of flows for training the U-net 504a. That is, FIG. 7A illustrates the learning phase. In the learning phase, the X-ray image 502a is an example of input image data and the X-ray image 508a is an example of the second medical image data.

Note that the learning phase is not necessarily distinguished from the inference phase. For example, after prescribed training for the U-net 504a has been completed, and after the use of the trained U-net 504a (inference phase) has started, the X-ray image 508a output from the U-net 504a may be used as training data to train the U-net 504a further. In this case, the X-ray image 502a is an example of input image data and also an example of the first medical image data. The X-ray image 508a is both the second medical image data and an example of a medical image with higher image quality than that of the first medical image data.

In FIG. 7A, an X-ray image 506a is an example of the wanted image (positive sample) 506 shown in FIG. 6 and also an example of the fourth medical image data. The X-ray image 506a is used as "Positive" data in training the U-net 504a using contrastive learning. That is, the X-ray image 506a is input as positive label data (Clear Image) with high image quality in the computation of L1Loss 612a and the encoding processing by the encoder 614a.

The x-ray image 510a is an example of the unwanted image (negative sample) 510 shown in FIG. 6 and is also an example of the third medical image data. The X-ray image 510a is used as "Negative" data in training the U-net 504a using contrastive learning. That is, the X-ray image 510a is input as negative label data (Hazy Image) with low image quality in the encoding processing by the encoder 614a.

The X-ray image 502a shown in FIG. 7A is data of low image quality with blurriness and noise. For example, the X-ray image 502a is a clinical image collected under low-dose X-ray conditions (low-dose image) or an image with simulated blurriness or noise added. The X-ray image 508a is data output from the U-net 504a that has received the input of the X-ray image 502a, and has reduced blurriness and noise compared to the X-ray image 502a.

The X-ray image 510a is used as "Negative" data in contrastive learning, and it is preferable that the X-ray image 510a have lower image quality than the X-ray image 508a, which is "Anchor" data. For example, the X-ray image 510a is data with more blurriness than the X-ray image 508a. To give an example, the X-ray image 502a, which is the input image data to the U-net 504a, may be used as-is as the X-ray image 510a. To give another example, the X-ray image 510a is an image with less noise and more blurriness than the X-ray image 502a. An image with less noise and more blurriness than the X-ray image 502a can be acquired, for example, by smoothing the X-ray image 502a. Even in the middle of training, the blurriness in the X-ray image 502a, which the input image data, is reduced to some extent by the U-net 504a. Thus, by containing the same or more blurriness than the X-ray image 502a, the X-ray image 510a can contain more blurriness than the X-ray image 508a output from the U-net 504a. It is preferable that the X-ray image 506a used as "Positive" data have higher image quality than the X-ray image 508a.

The X-ray image 506a, the X-ray image 508a, and the X-ray image 510a are input to the encoder 614a to highlight differences between the images. Thus, the encoder 614a encodes the positive image, the image predicted by the trained model, and the unwanted negative image, and, for example, increases or decreases the weight on a specific feature, so as to highlight differences between the images. A contrastive loss 616a is then computed based on the various encoded images.

In FIG. 7A, the contrastive loss 616a is shown conceptually. That is, in FIG. 7A, the X-ray image 506a used as "Positive" data is indicated by a star symbol, the X-ray image 508a used as "Anchor" data is indicated by a diamond symbol, and the X-ray image 510a used as "Negative" data is indicated by a cross symbol. The distance between these symbols indicates the distance between images in an optimization space. For example, the more similar the features are between images, the closer the symbols are plotted to each other.

The contrastive loss 616a can then be acquired by optimizing the loss function so that the distance between the X-ray image 508a and the X-ray image 510a increases (push) and the distance between the X-ray image 508a and the X-ray image 506a decreases (pull). That is, the contrastive loss can be acquired based on the loss based on the second medical image data and the third medical image data and the loss based on the second medical image data and the fourth medical image data. Note that any specific mathematical expressions for acquiring the contrastive loss 616a can be adopted, and are not limited to those illustrated in the embodiment.

Furthermore, L1Loss 612a is computed based on the X-ray image 510a and the X-ray image 508a. L1Loss 612a is a positive loss indicating the difference between "Positive" data and "Anchor" data. As in the case shown in FIG. 6, L1Loss 612a is added to the contrastive loss 616a to generate a total loss. The U-net 504a can then be trained based on the total loss generated. That is, the trained model can be trained based on the total loss obtained by adding the positive loss based on the second medical image data and the fourth medical image data to the contrastive loss based on the loss based on the second medical image data and the third medical image data and the loss based on the second medical image data and the fourth medical image data.

In FIG. 7A, the contrastive loss 616a is computed while highlighting differences between the images using the encoder 614a. The contrastive loss 616a is computed to be not only closer to "Positive" data, but also away from "Negative" data. Here, by including blurriness in the X-ray image 510a, which is "Negative" data, the U-net 504a, which is updated using the contrastive loss 616a, can be grown to remove the blurriness. That is, according to the medical image processing method according to the first embodiment, it is possible to remove not only noise but also blurriness from medical images, thereby improving the image quality of medical images.

When training is performed based only on L1Loss 612a (positive loss), the neural network is trained to bring input data closer to "Positive" data. Such a trained neural network corresponds to a learned model in a conventional method. Although learned models in conventional methods contribute to improving the image quality of medical images, they tend to over-smooth the images and blurriness tends to remain. Although it is possible to perform control of specializing in blurriness removal without changing the conventional methods, deep learning algorithms are difficult to control and may fall into unwanted solutions.

In contrast, in FIG. 7A, the use of contrastive learning allows for the appropriate removal of unwanted textures, for example, noise and blurriness simultaneously. In FIG. 7A, the U-net 504a is updated by generating a total loss based on the contrastive loss 616a and L1Loss 612a. In other words, while the conventional learned model based only on a positive loss also shows some contribution to improving image quality, in FIG. 7A, the conventional learned model can be improved by inserting an additional contrastive loss term so that unwanted textures can be appropriately removed.

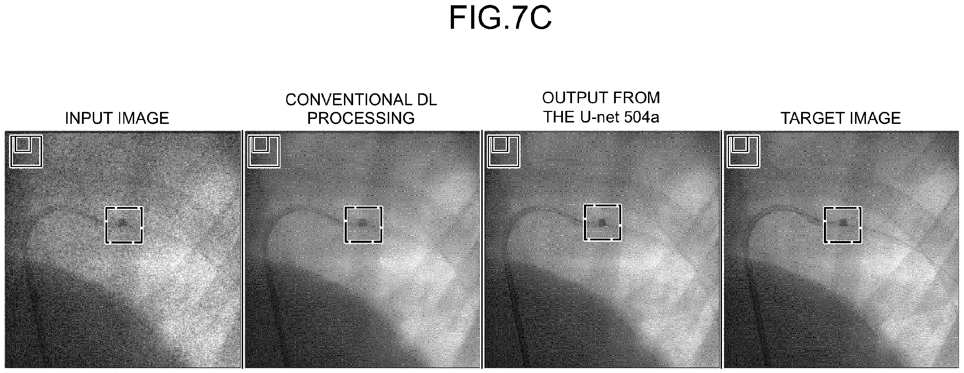

FIG. 7C illustrates an output from the U-net 504a trained in the flow of FIG. 7A. Also shown for comparison are an input image, an image from conventional DL processing, and a target image. The input image is input data to be processed by the U-net 504a or conventional DL processing, and is an image with noise and blur. For example, the input image is generated by adding noise and blurriness to the target image.

As shown in FIG. 7C, when the conventional DL processing is applied to the input image, granular noise can be removed, but it is excessively smoothed, resulting in fuzzy edges. In contrast, when the input image is processed by the U-net 504a, noise is removed and blurriness is suppressed to obtain a sharp result close to the target image.

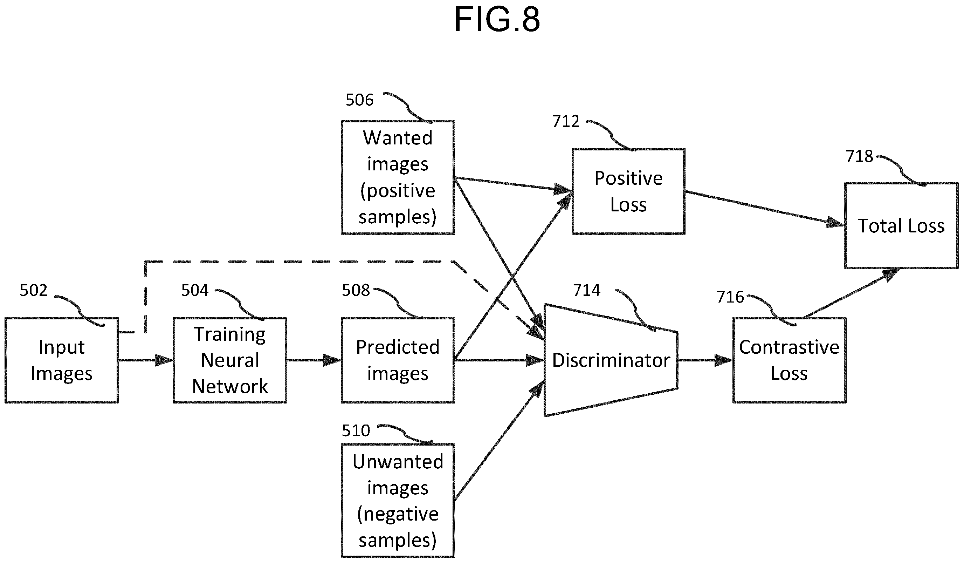

FIG. 8 is a flow diagram for a method of training a neural network by a contrastive loss method that includes a discriminator that is the inverse of the contrastive loss, in accordance with an exemplary aspect of the disclosure. In one embodiment, the contrastive loss 716 is determined based on a trainable discriminator 714. The discriminator 714 is a neural network that is trained based on a loss that is the inverse of the contrastive loss 716. The neural network 504 is trained based on a sum of the positive loss and the contrastive loss. The discriminator 714 is trained together with a neural network 504. In one iteration, discriminator 714 is fixed and then the neural network 504 is tunable, and then in the next iteration, NN 504 is fixed and then discriminator 714 is under training. That being said, discriminator 714 and the neural network 504 are trained alternatively. As the discriminator neural network 714 is trained, it enhances differences in negative samples from among the predicted image, the unwanted images, and the wanted image. The contribution to negative loss will be larger for negative samples. That is, the contrastive learning according to the embodiment may include training a discriminator on the inverse of a contrastive loss using, as input to the discriminator, positive images, images predicted by a trained model, and the negative label data.

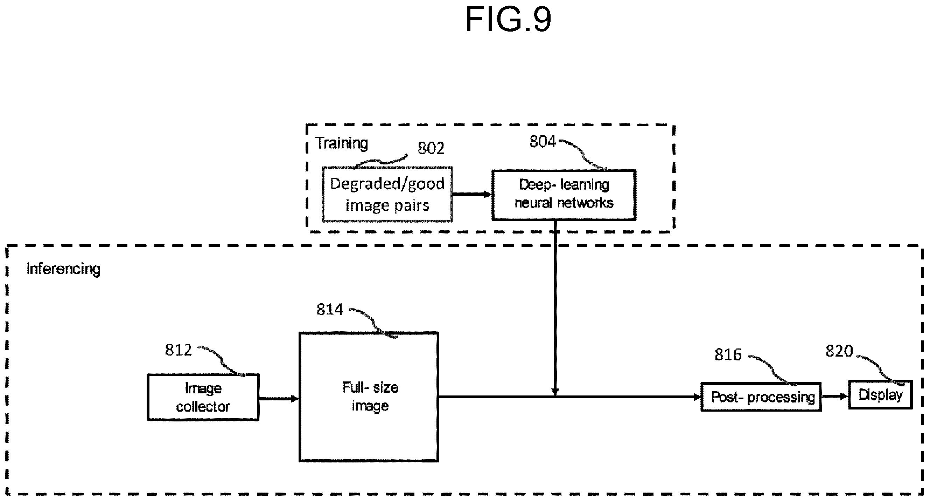

FIG. 9 is a flow diagram for deep-learning based image restoration, in accordance with an exemplary aspect of the disclosure. In order to perform inferencing, the trained neural network is used to output a predicted image based on an input image 814. The input image 814 can be a full-size image obtained from a storage device, as an image collector 812. The predicted image can be post processed in a post processor 816 in order to be displayed on a display device 820. The neural network is trained on unwanted and positive image pairs 802 using an embodiment of the deep learning method 804. For example, the acquisition function 111 shown in FIG. 1 may receive, as the first medical image data, X-ray fluoroscopy image data from a sequence of fluoroscopy images obtained by the image collector 812.

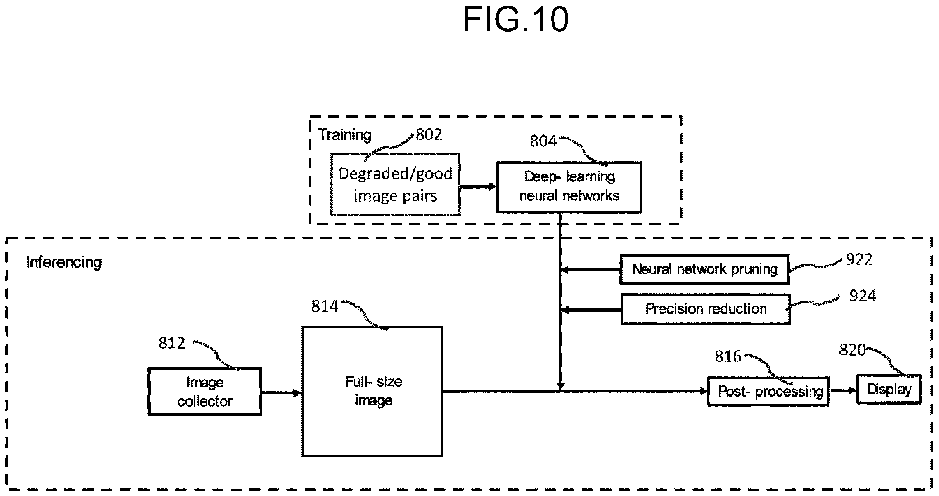

FIG. 10 is a flow diagram for deep-learning based image restoration that includes pruning and precision reduction for real-time inferencing, in accordance with an exemplary aspect of the disclosure. In order to speed up inferencing, the trained neural network may be pruned by a neural network pruning component 922 to remove unnecessary weighted connections, such as weighted connections that are below a predetermined value. The trained neural network may be further subject to precision reduction by a precision reduction component 924, either due to limitations of the processor, or to improve processing speed through simpler multiply-add computations. Precision reduction can be made by limiting the number of decimal places in weighted connections.

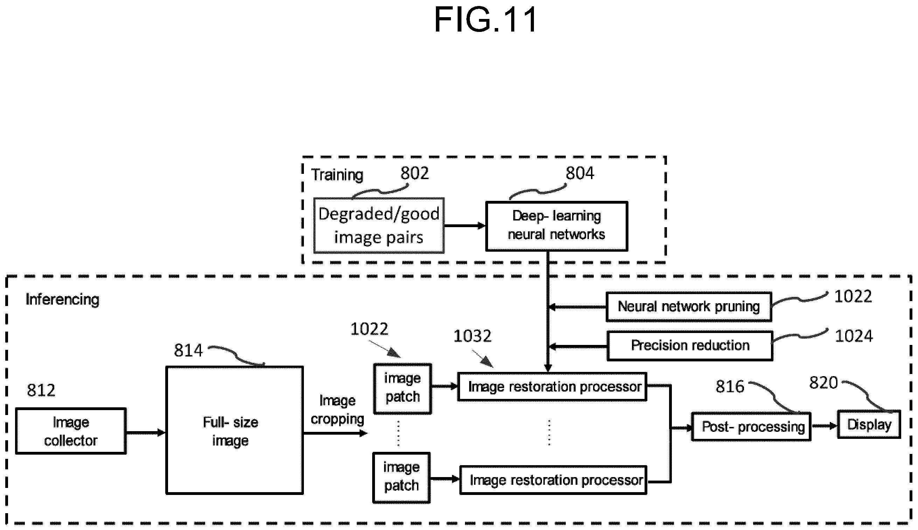

FIG. 11 is a flow diagram for deep-learning based image restoration that is implemented on multiple processors for real-time inferencing, in accordance with an exemplary aspect of the disclosure. In order to further speed up inferencing and make use of a processor with multiple cores, the input image 814 may be divided into multiple image patches 1022 by an image pre-processor, and the image patches are fed to different image restoration processors 1032 for inferencing to generate restored image patches. The different image restoration processors 1032 can be configured based on the deep learning network 804 that has been trained using an embodiment of the deep learning method. The deep learning network 804 can be pruned by a pruning component 1022 and be subject to precision reduction by a precision reduction component 1024. The different image processors can be configured to run in parallel. The image post-processor 816 can piece together the restored image patches to output a restored full image to the display device 820.

The disclosed embodiments of deep learning produce sharper images with clearer edges and more accurate texture without over-smoothing. The contrastive learning of the disclosed embodiments enables improved control of image quality compared to simply relying on good image quality as a learning criteria. The contrastive loss function explicitly discourages production of output data similar to the negative training data. To ensure better correlation between positive training data and negative training data, unwanted images can either be collected from clinical settings or can be obtained using simulation.

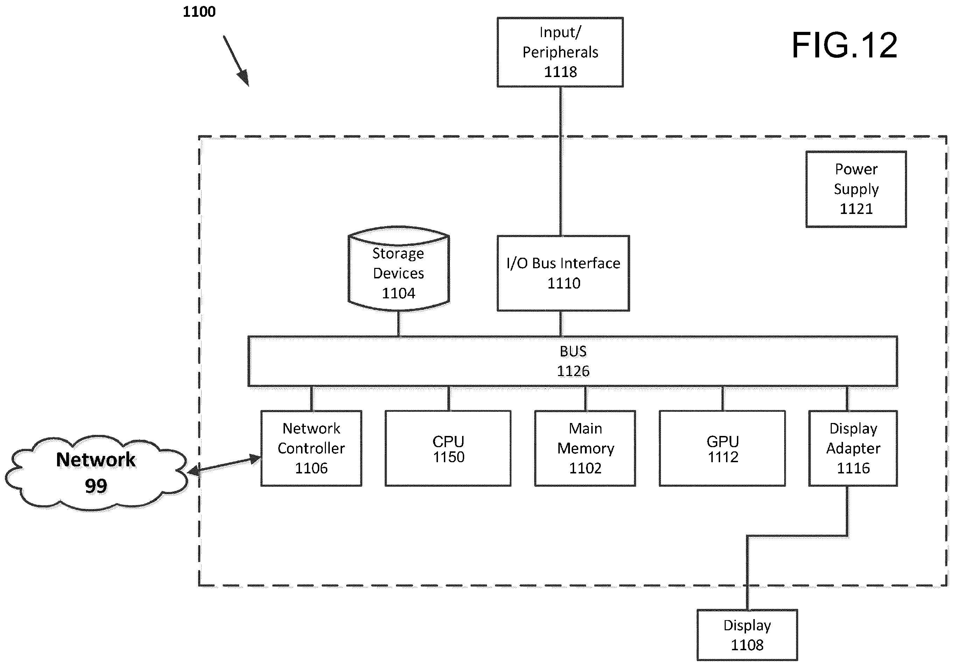

FIG. 12 is a block diagram illustrating an example computer system 1100 for implementing the machine learning training and inference methods according to an exemplary aspect of the disclosure. In a non-limiting example, the computer system 1100 can be an AI workstation running an operating system, for example Ubuntu Linux OS, Windows, a version of Unix OS, or Mac OS. The computer system 1100 can include one or more central processing units (CPU) 1150 having multiple cores. The computer system 1100 includes a plurality of GPUs 1112. For example, the computer system 1100 includes a graphics board having the GPUs 1112 each including a GPU memory. The GPU 1112 can perform many of the mathematical operations of the disclosed machine learning methods. For example, a combination of the CPU 1150, which is a processing core, and the GPU 1112 implements processing circuitry that executes the above described embodiments. The computer system 1100 includes main memory 1102, typically random access memory RAM, which contains the software being executed by the CPUs 1150 and GPUs 1112, as well as a non-volatile storage device 1104 for storing data and the software programs. Several interfaces for interacting with the computer system 1100 may be provided, including an I/O Bus Interface 1110, Input/Peripherals 1118 such as a keyboard, touch pad, mouse, Display Adapter 1116 and one or more Displays 1108, and a Network Controller 1106 to enable wired or wireless communication through a network 99. The interfaces, memory and processors may communicate over the system bus 1126. The computer system 1100 includes a power supply 1121, which may be a redundant power supply.

In one embodiment, the computer system 1100 includes a mult-core CPU and a graphics card by NVIDIA, in which the GPUs have multiple cores. In one embodiment, the computer system 1100 may include a machine learning engine such as the GPU 1112.

The above-described hardware description is a non-limiting example of corresponding structure for performing the functionality described herein.

Although X-ray images have been described as an example of medical images, the embodiments described above can be applied to medical images other than X-ray images. For example, ultrasound images may be used as the second medical image data, the third medical image data, and the fourth medical image data described above, and training using contrastive learning may be performed to generate a trained model. In addition, an ultrasound image may be input to the trained model as the first medical image data described above, and an ultrasound image with higher image quality than that of the input ultrasound image may be output from the trained model.

As in the case of X-ray images, there are no restrictions on the type of ultrasound image. For example, the ultrasound image may be B-mode data or Doppler data. The ultrasound image may be a two-dimensional image or a three-dimensional image. Alternatively, the ultrasound image may be a four-dimensional image collected continuously in the time direction. In addition to X-ray and ultrasound images, the embodiments described above can be applied to any type of medical images where noise or blurriness in the images is an issue.

Numerous modifications and variations of the present disclosure are possible in light of the above teachings. It is therefore to be understood that within the scope of the appended claims, the invention may be practiced otherwise than as specifically described herein.

According to at least one embodiment described above, the image quality of medical images can be improved.

Although several embodiments of the present invention have been described, these embodiments are presented as examples and are not intended to limit the scope of the invention. These embodiments can be implemented in various other forms, and various omissions, substitutions, and changes can be made without departing from the scope of the invention as defined by the appended claims.

**Claims**

1. A medical image processing method, comprising:

   inputting first medical image data (402) to a trained model (404, 504, 504a, 804); and
   outputting, from the trained model (404, 504, 504a, 804), a medical image having an image quality higher than an image quality of the first medical image data,
   wherein the trained model (404, 504, 504a, 804) was trained using contrastive learning using second medical image data (508) as input data, third medical image data (304, 308, 510) and fourth medical image data (302, 306, 506) as label data, the third medical image data (304, 308, 510) being negative label data having worse image quality than the fourth medical image data (302, 306, 506), and the fourth medical image data (302, 306, 506) being positive label data having better image quality than the second medical image data (508).

2. The medical image processing method of claim 1, wherein the third medical image data (304, 308, 510) is medical image data with blurriness.

3. The medical image processing method of either of claims 1 or 2, wherein the fourth medical image data (302, 306, 506) is medical image data with less noise and blurriness than the second medical image data (508).

4. The medical image processing method of any preceding claim, wherein the second medical image data (508) is image data with noise and blurriness, and the fourth medical image data (302, 306, 506) is image data with less noise and less blurriness than the second medical image data (508).

5. The medical image processing method of any preceding claim, wherein the fourth medical image data (302, 306, 506) is a high-dose image and the third medical image data (304, 308, 510) is a low-dose image.

6. The medical image processing method of any preceding claim, wherein

   the second medical image data (508) is data output from the trained model that has received an input of input image data, and
   the third medical image data (304, 308, 510) is data based on the input image data.

7. The medical image processing method of any preceding claim, wherein the third medical image data (304) is generated by adding at least one of: simulated blurriness; and noise, to the fourth medical image data (302, 306, 506).

8. The medical image processing method of any preceding claim, wherein the trained model (404, 504, 504a, 804) is trained based on a total loss obtained by adding a positive loss based on the second medical image data (508) and the fourth medical image data (302, 306, 506) to a contrastive loss based on a loss based on the second medical image data and the third medical image data and a loss based on the second medical image data (508) and the fourth medical image data (302, 306, 506).

9. The medical image processing method of any preceding claim, wherein the contrastive learning uses a negative loss function term to learn from unwanted negative images used for the third medical image data (304, 308, 510).

10. The medical image processing method of claim 9, wherein the contrastive learning includes encoding positive images, images predicted by the trained model (404, 504, 504a, 804), and the unwanted negative images so as to increase or decrease a weight for specific features.

11. The medical image processing method of any of claims 1 to 7, wherein the contrastive learning includes training a discriminator (714) on an inverse of the contrastive loss using, as input to the discriminator (714), positive images, images predicted by the trained model, and the negative label data.

12. An X-ray diagnosis apparatus comprising processing circuitry (110, 214, 1150, 1112) configured to: input first medical image data to a trained model (404, 504, 504a, 804); and output, from the trained model (404, 504, 504a, 804), a medical image having an image quality higher than an image quality of the first medical image data, wherein the trained model (404, 504, 504a, 804) was trained using contrastive learning using second medical image data as an input (508), and third medical image data (304, 308, 510) and fourth medical image data (302, 306, 506) as label data, the third medical image data (304, 308, 510) being negative label data having worse image quality than the fourth medical image data (302, 306, 506), and the fourth medical image data (302, 306, 506) being positive label data having better image quality than the second medical image data (508).

13. The X-ray diagnosis apparatus of claim 12, wherein the processing circuitry (110, 214, 1150, 1112) is further configured to receive, as the first medical image data, X-ray fluoroscopy image data from a sequence of fluoroscopy images obtained by an image collector.

14. The X-ray diagnosis apparatus of either of claims 12 or 13, wherein the processing circuitry (110, 214, 1150, 1112) is further configured to: remove, from the trained model (404, 504, 504a, 804), weighted connections that are below a predetermined value; and reduce a precision of the weighted connections of the trained model (404, 504, 504a, 804).

15. A method of generating a trained model, comprising:

   acquiring first training image data (508), second training image data (304, 308, 510), the second training image data (304, 308, 510) being unwanted negative image data having worse image quality than third training image data (302, 306, 506), and the third training image data (302, 306, 506), the third training image data (302, 306, 506) being wanted positive image data having better image quality than the first training image data (508); and
   training the neural network model (404, 504, 504a, 804) using contrastive learning using the first training image data (508) as input data and the second (304, 308, 510) and third (302, 306, 506) training image data as label data, wherein
   the contrastive learning includes a negative loss term for the neural network model to learn from the unwanted negative image data and a positive loss term for the neural network model to learn from the wanted positive image data.

# FIG.1

X-RAY DIAGNOSTIC APPARATUS (100)

- IMAGE DATA GENERATING CIRCUITRY
- STORAGE
- IMAGE PROCESSING CIRCUITRY
- DISPLAY

PROCESSING CIRCUITRY (110)
- ACQUISITION FUNCTION — 111
- SETTING FUNCTION — 112
- CONTROL FUNCTION — 113

- X-RAY DETECTOR
- C-ARM ROTATING/MOVING MECHANISM
- TABLETOP MOVING MECHANISM
- COLLIMATOR
- X-RAY TUBE
- C-ARM/TABLETOP MECHANISM CONTROL CIRCUITRY
- COLLIMATOR CONTROL CIRCUITRY
- HIGH VOLTAGE GENERATOR
- INPUT CIRCUITRY

P

# FIG.2

CURRENT REGULATOR — 208

HIGH VOLTAGE GENERATOR — 209

SYSTEM CONTROLLER — 210

RECONSTRUCTION CIRCUITRY — 214

STORAGE — 212

DISPLAY — 216

INPUT CIRCUITRY — 215

PREPROCESSING CIRCUITRY — 206

200

201

208 SLIP RING

207

ROTATING UNIT

S

RA

202

X-RAY DETECTOR

203

DATA ACQUISITION CIRCUITRY (DAS)

204

NONCONTACT DATA TRANSMITTER — 205

211

EP 4 428 808 A1

# FIG.3A

302

Simulated
wanted
images
(positive
samples)

304

Simulated
unwanted
images
(negative
samples)

310

Training
workflow

# FIG.3B

306

Clinical
wanted
images
(positive
samples)

308

Clinical
unwanted
images
(negative
samples)

310

Training
workflow

EP 4 428 808 A1

402

**X-ray image data** →[ NN ] 404

## FIG.4A

402
**Projection data** → 404 [ NN ] → 406 [ **Corrected Projection data** ] → 408 [ **Reconstruction** ] → 410 [ **Corrected 3D volume** ]

## FIG.4B

402
**Projection data** → 414 [ **Reconstruction** ] → 412 [ **3D volume** ] → 404 [ **NN** ] → 416 [ **Corrected 3D volume** ]

## FIG.4C

EP 4 428 808 A1

# FIG.5

EP 4 428 808 A1

# FIG.6

EP 4 428 808 A1

FIG.7A

FIG.7B

EP 4 428 808 A1

# FIG.7C

INPUT IMAGE | CONVENTIONAL DL PROCESSING | OUTPUT FROM THE U-net 504a | TARGET IMAGE

# FIG.8

# FIG.9

EP 4 428 808 A1

# FIG.10

EP 4 428 808 A1

# FIG.11

EP 4 428 808 A1

FIG.12

Europäisches
Patentamt
European
Patent Office
Office européen
des brevets

# EUROPEAN SEARCH REPORT

**Application Number**

EP 24 16 2444

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | TANG YUFEI ET AL: "CCN-CL: A content-noise complementary network with contrastive learning for low-dose computed tomography denoising", COMPUTERS IN BIOLOGY AND MEDICINE, NEW YORK, NY, US, vol. 147, 20 June 2022 (2022-06-20), XP087113707, ISSN: 0010-4825, DOI: 10.1016/J.COMPBIOMED.2022.105759 [retrieved on 2022-06-20] | 1,5,6, 8-10,12, 15 | INV. G06T5/60 |
| Y | * abstract * <br> * sections 2.3, 4 * <br> * figure 1 * <br> ----- | 2-4,7, 11,13,14 | |
| X | MINGHAN FU ET AL: "Total-Body Low-Dose CT Image Denoising using Prior Knowledge Transfer Technique with Contrastive Regularization Mechanism", ARXIV.ORG, CORNELL UNIVERSITY LIBRARY, 201 OLIN LIBRARY CORNELL UNIVERSITY ITHACA, NY 14853, 1 December 2021 (2021-12-01), XP091112891, * abstract * <br> * sections III.B, III.C * <br> * figure 1 * <br> ----- <br> -/-- | 1,12,15 | |

| | | | TECHNICAL FIELDS SEARCHED (IPC) |
|---|---|---|---|
| | | | G06T |

The present search report has been drawn up for all claims

| Place of search | Date of completion of the search | Examiner |
|---|---|---|
| Munich | 22 July 2024 | Engels, Angela |

Europäisches
Patentamt

European
Patent Office

Office européen
des brevets

# EUROPEAN SEARCH REPORT

**Application Number**

EP 24 16 2444

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | JAN FENG-KAI ET AL: "Contrastive Learning Aided Single Image Deblurring", 2022 IEEE INTERNATIONAL CONFERENCE ON CONSUMER ELECTRONICS-ASIA (ICCE-ASIA), IEEE, 26 October 2022 (2022-10-26), pages 1-3, XP034235457, DOI: 10.1109/ICCE-ASIA57006.2022.9954660 [retrieved on 2022-11-28] * abstract * * section 2 * * figure 1 * | 2-4 | |
| Y | US 2022/414832 A1 (HU YI [US] ET AL) 29 December 2022 (2022-12-29) * abstract * * paragraph [0001] - paragraph [0005] * * paragraph [0057] * * paragraph [0065] * * claim 1 * | 2-4,7, 13,14 | |
| Y | US 2023/071693 A1 (KANG EUNHEE [KR] ET AL) 9 March 2023 (2023-03-09) * abstract * * paragraph [0003] * * paragraph [0112] - paragraph [0122] * * figures 2B, 6 * | 11 | **TECHNICAL FIELDS SEARCHED (IPC)** |

The present search report has been drawn up for all claims

| Place of search | Date of completion of the search | Examiner |
|---|---|---|
| Munich | 22 July 2024 | Engels, Angela |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

......................................................................

& : member of the same patent family, corresponding
   document

EPO FORM 1503 03.82 (P04C01)

**EP 4 428 808 A1**

## ANNEX TO THE EUROPEAN SEARCH REPORT
## ON EUROPEAN PATENT APPLICATION NO.

EP 24 16 2444

This annex lists the patent family members relating to the patent documents cited in the above-mentioned European search report.
The members are as contained in the European Patent Office EDP file on
The European Patent Office is in no way liable for these particulars which are merely given for the purpose of information.

22-07-2024

| Patent document cited in search report | Publication date | Patent family member(s) | Publication date |
|---|---|---|---|
| US 2022414832 A1 | 29-12-2022 | EP 4123572 A2 | 25-01-2023 |
| | | JP 2023004941 A | 17-01-2023 |
| | | US 2022414832 A1 | 29-12-2022 |
| US 2023071693 A1 | 09-03-2023 | KR 20230032717 A | 07-03-2023 |
| | | US 2023071693 A1 | 09-03-2023 |

EPO FORM P0459

For more details about this annex : see Official Journal of the European Patent Office, No. 12/82